(19) **Europäisches Patentamt**
**European Patent Office**
**Office européen des brevets**

(11) **EP 4 732 804 A2**

## (12) EUROPEAN PATENT APPLICATION

(43) Date of publication:
**29.04.2026 Bulletin 2026/18**

(21) Application number: **25211088.7**

(22) Date of filing: **24.10.2025**

(51) International Patent Classification (IPC):
**A61B 34/20** (2016.01)     **A61B 5/06** (2006.01)
**G01R 33/00** (2006.01)     **G01R 33/02** (2006.01)
**G01R 33/09** (2006.01)

(52) Cooperative Patent Classification (CPC):
**A61B 34/20; A61B 5/062; G01R 33/0005;
G01R 33/0023; G01R 33/0082; G01R 33/091;**
A61B 2034/2051; A61B 2090/0818; G01R 33/028

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **25.10.2024 US 202463711833 P**

(71) Applicant: **RADWAVE TECHNOLOGIES INC.
St. Paul, Minnesota 55114 (US)**

(72) Inventors:
• **MORGAN, Sean M.
Golden Valley, 55422 (US)**

• **KOYRAKH, Lev A.
Plymouth, 55442 (US)**
• **YOUNG, Joseph
White Bear Lake, 55110 (US)**
• **WIDULE, Matthew
River Falls, 54022 (US)**
• **WEISENBERGER, Michael R.
Rogers, 55374 (US)**
• **BROWN, Andrew E.
Minneapolis, 55414 (US)**

(74) Representative: **Mathys & Squire
32 London Bridge Street
The Shard
London SE1 9SG (GB)**

## (54) MAGNETO-RESISTIVE AND COIL SENSOR TRACKING SYSTEM

(57) An electromagnetic tracking system includes an adjustable source of electricity, a magneto-resistive (MR) sensor coupled to the adjustable source of electricity and configured to couple to a device for tracking a position of the device, and a controller coupled to the adjustable source of electricity and the MR sensor to adjust an amount of electricity provided to the MR sensor by the adjustable source of electricity. The electromagnetic tracking system is configured to track MR sensors and coil-based sensors simultaneously using one or more input channels on the controller.

FIG. 1

EP 4 732 804 A2

**Description**

**RELATED APPLICATION**

This application claims priority to United States Provisional Application serial number 63/711,833 (entitled System, method and apparatus to track magneto-resistive sensors with electromagnetic tracking systems, filed October 25, 2024) which is incorporated herein by reference.

**BACKGROUND**

Electromagnetic (EM) tracking systems track the position and orientation of sensors located within, for example, surgical tools within a spatial volume of electromagnetic fields generated by the tracking system. The electromagnetic fields avoid ionizing radiation to users and patients, in the context of medical applications. Electromagnetic tracking systems work by generating multiple geometrically distinct electromagnetic fields by different antenna elements, which are sensed by electromagnetic field sensors. A small EM sensor is often embedded in the tip or body of a medical tool that is placed within the field. Signals from the sensors are used to compute the sensors' positions and orientations. The number of required electromagnetic fields depends on the type of sensors. Sensors represent a critical part of electromagnetic (EM) tracking technology. Currently, commercially available EM tracking systems use coil sensors. A coil sensor is a coil of wire which detects changes in the magnetic field and generates a voltage based on its position and orientation. Coil sensors can be relatively large, expensive and difficult to mass produce in large quantities, requiring a significant amount of manual labor. It is also challenging to embed coil sensors into medical tools due to significant space requirements, which creates a barrier to the adoption of the EM tracking. Additionally, coil sensors require relatively high frequency electromagnetic fields, as their output signal voltage is directly proportional to the field frequency, which, in turn, makes them susceptible to electromagnetic field distortions caused by eddy currents.

Accordingly, there is a need to track sensors which can operate at zero (DC) or low frequency magnetic fields, which are sufficiently small to be embedded within various instruments and can be inexpensively produced in large quantities.

**SUMMARY**

An electromagnetic tracking system and method tracks magnetoresistive (MR) sensors . The electromagnetic tracking system includes an adjustable source of electricity and an MR sensor coupled to the adjustable source of electricity. The MR sensor is configured to couple to a device for tracking a position and orientation of the device. A controller provides the adjustable source of electricity to the MR sensor. An electromagnetic field generator provides the fields necessary for the MR sensor to generate an input signal to the controller. The controller receives the input signal and uses it to determine the position and orientation of the MR sensor within the electromagnetic field.

To increase the dynamic range of the MR sensor, its sensitivity is adjusted by varying the input voltage based on its signal strength. Coil sensors can be used along with MR sensors and can be connected to different or the same input channels of the controller.

**BRIEF DESCRIPTION OF THE DRAWINGS**

These and other features, aspects, and advantages of the present invention will become better understood when the following detailed description is read with reference to the accompanying drawings in which like characters represent like parts throughout the drawings, wherein:

FIG 1 is a diagram of an electromagnetic tracking system in the clinical context according to an example embodiment.
FIG. 2A, 2B, 2C, and 2D illustrate MR sensor construction and assembly examples that can interface to a controller according to an example embodiment.
FIG. 3 illustrates a system that includes an MR sensor interfacing to a controller according to an example embodiment.
FIG. 4 illustrates a timing for energizing different antenna elements in time-division-multiplexing scheme for tracking MR sensors according to an example embodiment.
FIG. 5 illustrates a system that includes an MR sensor and a coil sensor interfacing to a controller through different input channels according to an example embodiment.
FIG. 6 illustrates a system that includes an MR sensor and a coil sensor interfacing to a controller through the same input channel according to an example embodiment.
FIG. 7 illustrates signal processing flow chart for separation of input signals from MR sensor and a coil sensor interfacing to a controller through the same input channel using frequency-division-multiplexing according to an example embodiment.

FIG. 8 illustrates a flow chart for automatic MR sensor tracking range adjustment according to an example embodiment.

FIG. 9 illustrates a block schematic diagram of a computer system to implement a controller for MR sensor-based position sensing and for performing methods and algorithms according to example embodiments.

## DETAILED DESCRIPTION

In the following description, reference is made to the accompanying drawings that form a part hereof, and in which is shown by way of illustration specific embodiments which may be practiced. These embodiments are described in sufficient detail to enable those skilled in the art to practice the invention, and it is to be understood that other embodiments may be utilized and that structural, logical and electrical changes may be made without departing from the scope of the present invention. The following description of example embodiments is, therefore, not to be taken in a limited sense, and the scope of the present invention is defined by the appended claims.

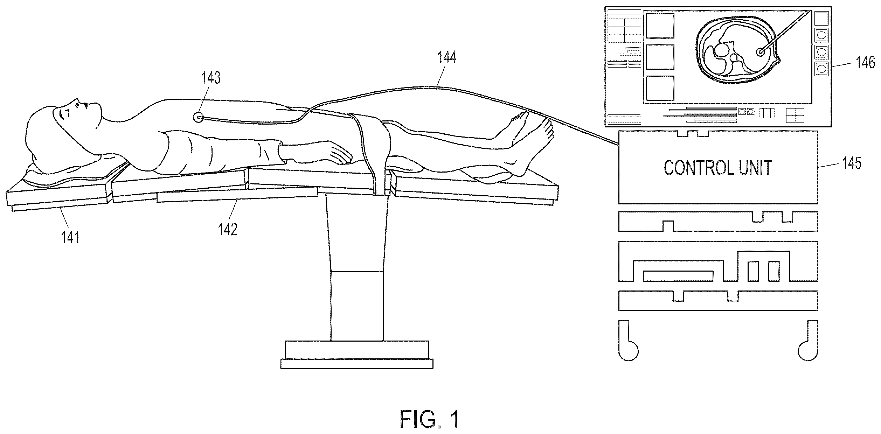

FIG. 1 is a diagram of an electromagnetic tracking system in a clinical context. An electromagnetic field generator (antenna) 142 comprising multiple antenna elements is placed under a patient's procedure area 143 and can be positioned around the patient as required by the medical procedure. A surgical tool 144 is placed inside the patient's body and is shown on a procedure application screen of a computer 146 overlayed on the anatomical map of the patient, allowing three-dimensional position and orientation navigation of the tool within the patient.

An electromagnetic field created by the antenna 142 generates voltage and/or current in one or more sensors coupled to the surgical tool 144 and connected to the inputs of the computerized data acquisition system known as Control Unit (CU) 145. CU 145 demodulates and conditions sensor inputs and computes sensor position and orientation (P&O).

The P&O information is then transferred to a computer 146 for tracking and displaying the surgical tool position within the patient in procedure area 143 corresponding to a navigation space of the field generator antenna 142.

The antenna 142 creates a plurality of N electromagnetic fields with different spatial geometries, $\overrightarrow{B_\alpha}$ (x,y,z), where $\alpha$=1,...N, where N is the number of antenna elements, and (x,y,z) represent a point in space around the antenna 142. In practical applications, the number of antenna elements, N varies between 3 and over 12.

Each sensor, MR or coil, is approximated as a point-like magnetic dipole having coordinate (x,y,z) and vector direction $\overrightarrow{m}$, which denotes the orientation of the sensor.

A single MR or coil sensor can provide only 5 degrees of freedom (DOF) as it has only a single orientation vector. For many applications 6DOF tracking, which includes resolving an additional tool roll angle is required. To accomplish this, multiple MR or coil sensors having different mutual orientations are placed in proximity to each other and localized either independently or with various assumptions about their mutual locations and orientations.

Every sensor connected to the system generates its own set of input signals as raw waveforms, that after applying the appropriate signal processing techniques are represented by the N pickups $V_\alpha$. Various conventional signal processing techniques, such as sampling and/or demodulation, can be used to determine the pickups from the raw input waveforms, as described below. The pickups are used by the localization software within the control unit 145 to compute positions and orientations of the sensors.

In one embodiment, once pickups are available to the system, the sensor can be localized using a search algorithm, such as Levenberg-Marquardt. For a single dipole-like 5DOF sensor, the search algorithm is used to find the position (x,y,z) and orientation $\overrightarrow{m}$ of the sensor that minimizes the following error function:

$$error = \sum_{\alpha=1}^{N} \left( \overrightarrow{B_\alpha} (x,y,z) \cdot \overrightarrow{m} - V_\alpha \right)^2 + w_g \left( | \overrightarrow{m} | - g \right)^2, \qquad (1)$$

where the first term represents the position and orientation error, and the second term represents the constraint on the sensor gain $g$ which characterizes the strength or sensitivity of the sensor. The parameter $w_g$ is the gain term weight which can be chosen in the range between 0 and over 1000, depending on the specific tracking application. When $w_g = 0$, the gain is not constrained and is found as the result of the minimization of the first term. This may be useful when the sensor gain $g$ is not known beforehand. When the gain value $g$ is known prior to the sensor localization and $w_g > 0$, the gain value is constrained by the second term, which may help the algorithm to find a more precise solution to the problem when the gain value is available.

The Equation (1) is suitable for localizing a single 5DOF coil or MR sensor. With 6DOF sensors additional terms are added to the Equation (1) that describe similar constraints on the additional dipole-like sensors comprising the 6DOF sensor. In one embodiment, with the number of 5DOF dipoles comprising a 6DOF sensor equal to $S$ (which usually takes values of 2 or 3), the corresponding error function can be written as the sum of the individual errors for the dipole-like sensors:

$$error = \sum_{k=1}^{S} \left( \sum_{\alpha=1}^{N} \left( \vec{B_\alpha}(x^k, y^k, z^k) \cdot \vec{m^k} - V^k_{\alpha} \right)^2 + w_g \left( |\vec{m^k}| - g^k \right)^2 \right), \quad (1')$$

where $(x^k, y^k, z^k)$ denote the location of the individual dipoles, $\vec{m^k}$ are the dipole orientations, and $g^k$ are their gain values. Additional constraints describing relationships between the individual dipoles, such as orientation vector dot products or spatial vectors connecting different dipoles, can be added to the error function (1'). For example, if a dot product between two orientation vectors $\vec{m^k}$ and $\vec{m^j}$ is known to be $dot^{kj}$, the additional term $w_{dot}$ $(\vec{m^k} \cdot \vec{m^j} - dot^{kj})^2$, where $w_{dot}$ describes the corresponding weight that can be chosen in the range between 0 and over 1000, can be added to the error function (1') to further constrain the solution.

The additional constraints effectively reduce the number of variables that must be found from the equation making it potentially easier to solve.

In one embodiment, when the individual magnetic dipoles within the sensor are placed sufficiently close to each other (usually less than 1 mm or as required by the application), the positions $(x^k, y^k, z^k)$ may be described as a single value $(x, y, z)$ common to all dipoles, which would further reduce the number of variables and simplify the solution to the problem.

In the ideal case with no electromagnetic field distortions and environmental noise, the minimized error (1) or (1') would very close to zero, reflecting the exact mathematical relationship between the magnetic fields and the voltage they generate in the sensors.

Alternatively, the localization steps defined by the disclosed equations may be performed by any suitable computing means, including without limitation, Radwave Technologies Inc. localization software.

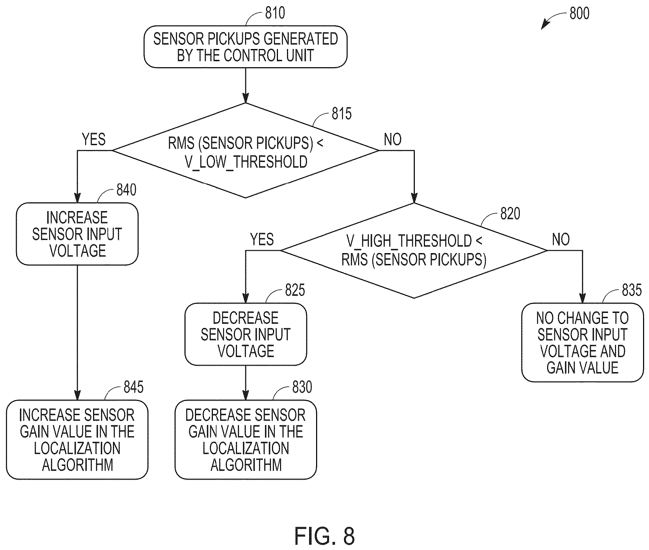

In another embodiment, the gain value $g$ of the MR sensor can be controlled by the input voltage to the sensor, as described below. In that case, the sensor gains in Equations (1) and (1') should be adjusted accordingly, as described below and in FIG. 8.

Currently, most electromagnetic tracking systems use coil sensors of different sizes. Relying on coil sensors creates multiple limitations. Such limitations are due to coil sensors being relatively large, expensive and difficult to mass produce in large quantities.

Over the last decades, new solid-state sensors have been developed based on the quantum magnetoresistance (MR) effects. MR sensors are based on a change in resistance induced by the application of an external magnetic field, such as tunneling (TMR) and Giant magnetoresistance (GMR).

With the current state of the art of semiconductor processing and magnetic material deposition techniques, solid-state MR sensors can be made sufficiently small (millimeter to sub-millimeter in size) for embedding into restricted volumes within medical devices while still maintaining a high sensitivity, less costly (one-tenth or less than the cost of coil-based sensors), and can be much easier to produce in large quantities due to their typical wafer based production methods. For example, a suitable solid-state MR sensor is the NVE Corporation ALT-Series TMR Analog Magnetometer Sensor, such as the ALT025-14E, which has a 1.1 x 1.1 mm DFN4 package and a linear operating field range of $\pm 10$ mT. This sensor also has a high sensitivity (up to 22.5 mV/V/mT typical for the ALT025-14E version) and a highly linear output of less than 1% of full scale. The ALT-Series TMR sensors are implemented in the Wheatstone bridge configuration and can operate at extremely low supply voltages, and the output signal is proportional to the supply. Additionally, the miniature size and lower cost of MR sensors would significantly expand their usage within additional tools requiring smaller and less expensive sensors. For example, multiple MR sensors could be placed along the length of a catheter in order to display the catheter's shape at a lower cost than a single coil-based sensor placed at the end of the catheter. Such shape sensing would allow users, such as surgeons, to know the locations of the entire length of the catheter, and not only the end point of the catheter. The user can be informed of the location of the entire catheter with respect to anatomy within the patient.

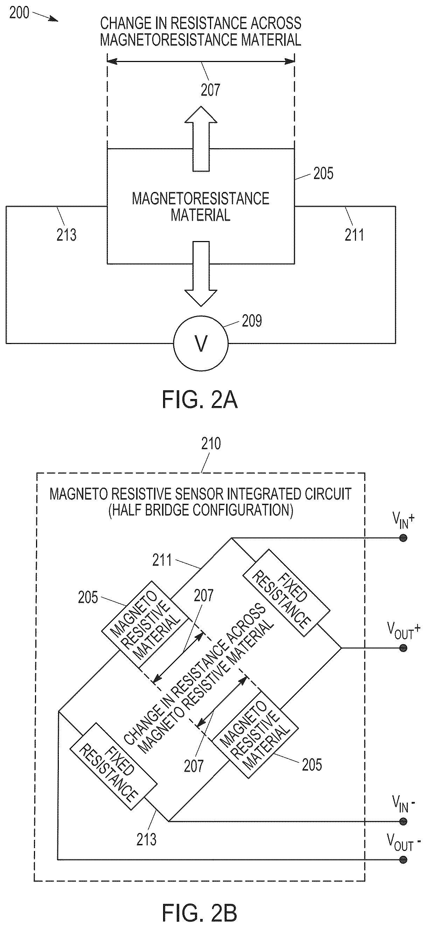

FIG. 2A is a schematic diagram of an MR sensor 200. MR sensor 200 includes a sensing element 205 formed of a magnetoresistive material. A change in resistance 207 across the sensing element 205 in response to a magnetic field is measured to provide an indication of a strength of the magnetic field. A voltage or current source 209 is coupled to the sensing element 205 via conductors 211 and 213.

FIG. 2B is a schematic diagram of an integrated circuit MR sensor 210. MR sensor 210 includes sensing element(s) 205 formed of a magneto resistive material that are built into a conventional Wheatstone bridge in a half bridge (two active sensors) configuration to aid in converting resistance changes to voltage changes as one example sensor integrated circuit embodiment. A Wheatstone bridge is known for this type of resistance to voltage conversion for a variety of active sensor types (magnetic field sensing, temperature sensing, strain gauges, etc.). Other Wheatstone bridge configurations are possible as well such as quarter bridge (single active sensor) or a full bridge (four active sensors) and the Wheatstone bridge can be implemented independently or separately from the sensing element(s) 205, but typically performs better when included as part of the integrated MR sensor 210. Sensing element(s) 205 may be deposited as a thin film of a magnetically sensitive alloy, such as Permalloy (a nickel-iron alloy) onto a substrate, such as a silicon substrate.

A change in resistance 207 across the sensing element(s) 205 in response to a magnetic field is measured to provide an indication of the strength of the magnetic field. A voltage or current source connected between $V_{in}+$ and $V_{in}-$ is

coupled to the sensing element(s) 205 via conductors 211 and 213.

Other layers may be added depending on the sensing element type, including a conductive layer (e.g., aluminum or gold) to form the electrical contacts, dielectric or isolating layers (e.g., silicon oxide) to separate the conductive layers and protect the MR sensor 210. In addition, other protection components such as Transient Voltage Suppression (TVS) diodes can be added internally to the integrated circuit to protect the MR sensor 210. The specific stack up on the substrate and any additional integrated electrical protection can vary by sensor manufacturer, but the basic connections to the MR sensor 210 (input power and output voltage) typically remain the same with a range a sensor power input requirements, sensor sensitivity specifications, linearity and sensor output voltage noise.

A tunnel barrier layer may be added for tunnel magnetoresistance (TMR) sensors to allow electrons to tunnel through the sensing element(s) 205.

The deposited layers are then structured to create the desired sensing element 205 geometry, such as a rectangle as shown, or other desired geometry. Laser target structuring can be used to create specific pathways for the current within the sensing element(s) 205.

Bond pads may be created on the substrate for making external electrical connections. These can then be connected to a lead frame or a printed circuit board using wire bonds. A protective passivation layer may be applied to the sensing element(s) 205 to shield it from environmental factors.

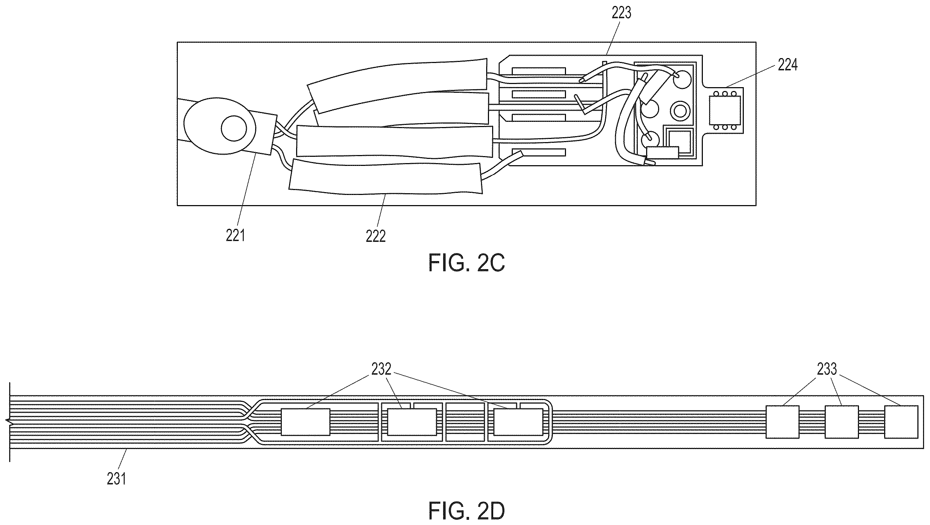

Prototype examples of MR sensors adapted for use with the electromagnetic tracking system are shown in FIGs. 2C and 2D. In FIG. 2C illustrates a 2.5x2.5 mm TMR (tunnel magnetoresistance) sensor integrated circuit with a built in Wheatstone bridge circuit 224 with high sensitivity is connected to a circuit board 223 containing electronics required for maintaining optimal sensing regimes and buffer amplifiers for signal conditioning and interfacing with control unit 45 in FIG 1 though connectors 222 and a cable 221 having the appropriate number of wires to supply the input voltage to the sensor 210 and the output differential voltage to the control unit 45.

As described above, a single MR sensor can only provide 5 degrees of freedom (DOF) as it has only a single orientation vector. To achieve 6DOF tracking, more sensors (at least one additional) are required that are placed at different angles to each other, as shown in FIG. 2D, where three 1.1 square millimeter miniature TMR sensors 233 having different mutual orientations are attached to the flexible circuit board, connected to correspondingly three different buffer amplifiers for signal conditioning 232, that are connected to the control unit through the flex cable 231, which is in turn connected to the cable similar to the cable 221 in FIG. 2C having the appropriate number of wires to supply the input voltage to and conduct the output signals from the three sensors.

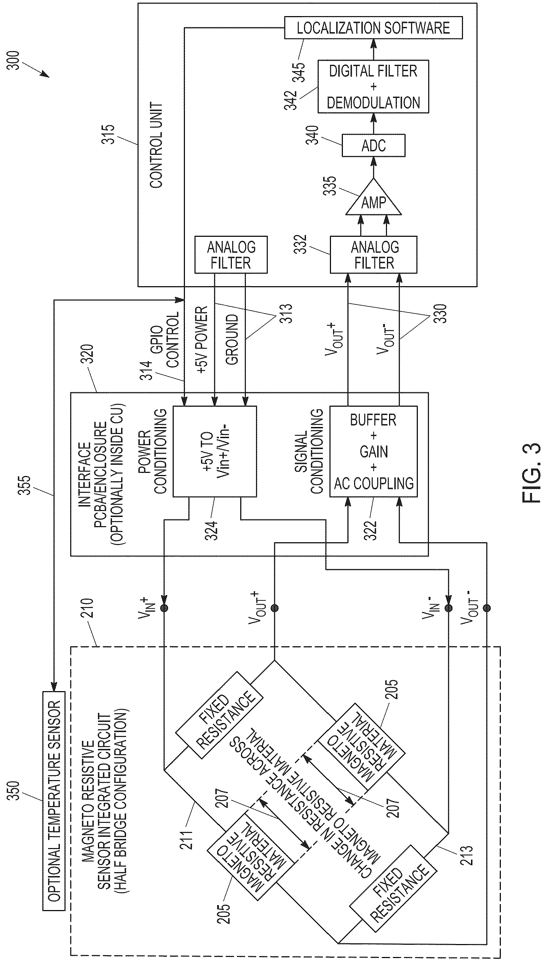

FIG. 3 is a block schematic diagram of an MR based sensing system 300. Reference numbers in FIG. 3 are consistent with reference numbers in FIG. 2 for like elements. Sensing system 300 includes an MR sensor 210 and a control unit 315 that provides input power on line 313 and an optional control line 314 to a printed circuit board assembly (PCBA) 320. PCBA 320 may be any type of structure that can support electrical components, such as a FR4 or ceramic printed circuit board with components such as amplifiers, filters and voltage regulators. The PCBA 320 is an optional way of connecting an MR sensor 210 to the control unit 315. The PCBA 320 includes signal conditioning 322 such as filters, amplifiers and buffers, and also power conditioning 324, such as digitally controlled voltage regulators.

The Wheatstone bridge within the MR sensor 210 provides a differential voltage output between Vout+ and Vout-. This voltage is coupled to the signal conditioning 322, which is then coupled to the control unit 305 via conductors 330.

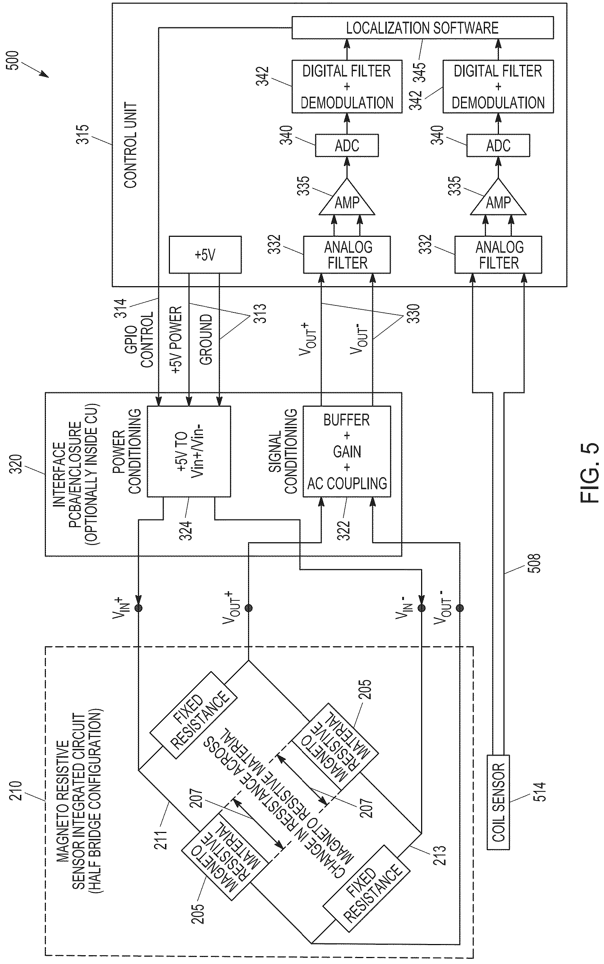

The Wheatstone bridge circuit can be integrated with the MR sensing elements 205 as shown by the MR sensor 210, and can also be implemented independently, as shown in FIG. 2B. Using the Wheatstone bridge for converting resistance into voltage allows connecting MR sensor 210 to the same input channels as a coil sensor as shown in FIG 5. Moreover, MR sensing elements 205 can be used with the same magnetic fields as coil sensors, which allows using the same signal processing techniques as are already available in the control unit 315 for tracking coil sensors. Control unit 315 contains input analog filters 332 for rejecting noise outside of tracking signal frequency bands, signal amplifier 335, analog-to-digital converter (ADC) 340 which is followed by digital signal processing including demodulation 342 which extracts signal amplitudes, or pickups, from the raw input waveforms. The signal amplitudes are processed by a localization engine 345 utilizing Equations (1) and (1') to provide sensor position and orientation information to a user. Alternatively, the localization may be performed by any suitable computing means, including without limitation, Radwave Technologies Inc. localization software.

MR sensing element-based sensors respond to the magnetic field strength rather than to a change (time derivative) in field strength which is detected by coil-based sensors. This different response enables the use of both types of sensors together since they respond to fields differently. This different response enables the use of a time-varying (AC) or constant (DC) magnetic field to localize MR sensing elements at the same time that an AC magnetic field is used to localize coil sensors. Both MR sensing elements and coil sensors may be co-located within a sensor device or located anywhere within the same navigation space without interfering with each other's sensing capabilities.

In one embodiment, shown in FIG. 3, a temperature sensor 350 may be thermally coupled to the MR sensor 210 to provide temperature information via a communication channel 355 to the control unit 315 to regulate the input voltage 313 or Vin+ and Vin- via the power conditioning unit 324 to improve temperature stability of the MR sensor 210. In one example, a temperature set point or a range (15°C -45°C) may be specified within control unit 315 implementing a conventional proportional/integral/derivative (PID) algorithm to control the MR sensor 210 input voltage using the measured temperature to ensure the MR sensor 210 output voltage remains constant for a constant magnetic field strength over a given temperature range. In addition, instead of requiring the temperature sensor 350 and the extra communication channel 355, the temperature stability of the MR sensor 210 could be provided by monitoring the voltages sensed by the MR sensor 210 from the plurality of antennas and then using the relative strengths produced from the different antennas instead of the absolute strengths to track the MR sensor. As the temperature goes up or down, all voltage strengths stemming from the plurality of antenna elements would change together as function of temperature, so by using the relative strengths, the dependence on the MR sensor 210 temperature would be minimized.

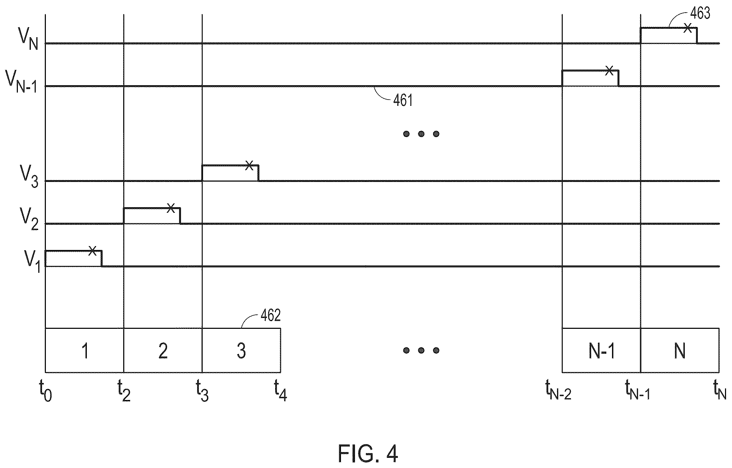

FIG. 4 is a set signal traces over time illustrating one embodiment of how a momentary DC magnetic field created by different antenna elements at different times in a time-division-multiplexing manner are utilized to create responses in the MR sensor that can be used for the sensor localization.

Each sensing element i=1, ... ,N is energized one by one within the time period 462 between times $t_{i-1}$ and $t_i$. After all antenna elements are energized, the process repeats. Since the MR sensor response is directly proportional to the magnetic field, by sampling the sensor output at the time points 463 denoted by x, the complete set of pickups can be produced for the sensor localization. By choosing sampling times away from the rising/falling edges of the magnetic field, where eddy currents are generated in the surrounding conductive materials by the varying magnetic fields sufficiently subside, the sensors pickups can be made insensitive to the electromagnetic interference caused by the eddy currents.

The same time-division-multiplexing scheme for antenna elements excitations can also be used with coil sensors. The raw waveforms from the coil sensors are processed to derive the signal amplitudes (pickups). This process involves steps such as integrating the raw waveforms around the rising and falling edges and then sampling the integrated values, a technique well-known in the art of electromagnetic tracking systems.

FIG. 5 illustrates a tracking system 500 that utilizes two separate control unit channels each having the same elements for processing signals from different types of sensors and will be described in further detail below. Reference numbers in FIG. 5 are consistent with reference numbers in FIG. 2 and FIG. 3 for like elements.

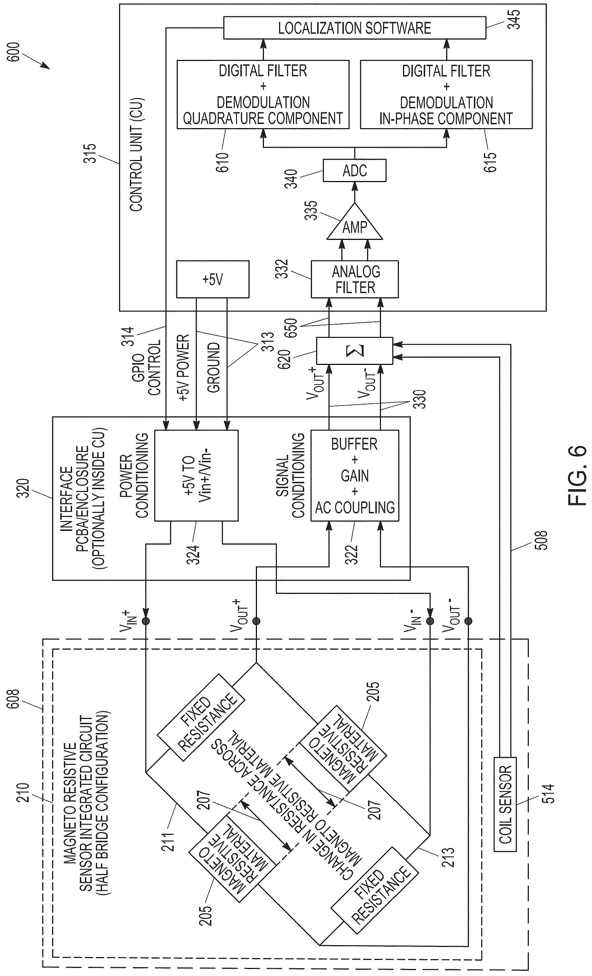

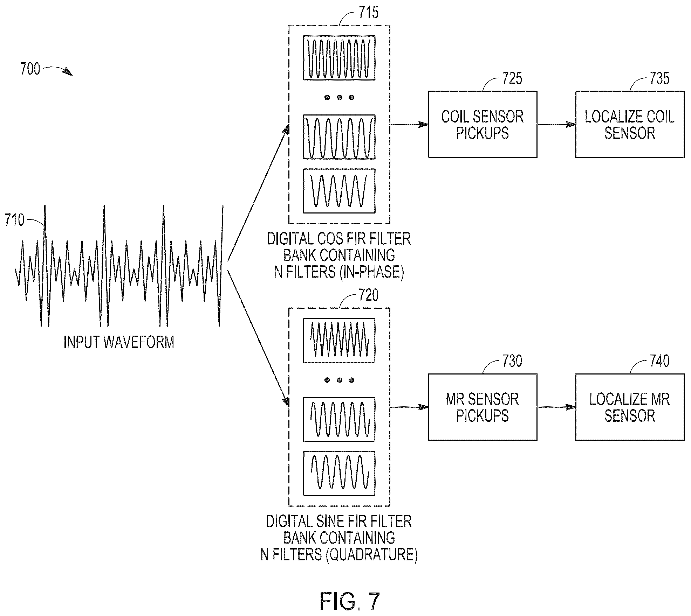

FIG. 6 illustrates a tracking system 600 for processing multiple sensor signals via a single control unit channel in a time-division-multiplexing or in a frequency-division-multiplexing manner. Reference numbers in FIG. 6 are consistent with reference numbers in FIG. 2, FIG. 3 and FIG. 5 for like elements. System 600 includes MR sensor 210 as well as a coil sensor element 514. Coil sensor element 514 already provides a voltage on conductors 508, so as opposed to being coupled to a Wheatstone bridge, voltage from coil sensor element 514 can be summed with the MR sensor 210 output voltage using a summing element 620 (amplifier or transformer), the output of which can be provided to the control unit 315 on conductors 650. This arrangement allows for an effective doubling of the number of sensors that can be used with control unit 315. Output from the ADC 340 includes both quadrature components and in-phase components processed respectively by a digital filter quadrature component 610 and digital filter in-phase component 615, both of which provide output to the localization software 345. The demodulation process is illustrated in more detail in FIG. 7.

In one embodiment, a hybrid sensor assembly indicated by the broken line 608 includes a coil-based sensor 514 and an MR sensor 210 that are co-located to create a hybrid 5DOF sensor 608.

In another embodiment the MR-based 210 sensor and coil-based 514 sensor forming the hybrid sensor 608 are orientationally aligned to produce 5DOF sensor position and orientation signals that have the benefit of the stronger coil-based sensor localized using the higher frequency magnetic fields along with the eddy currents distortion rejection benefits of the MR sensor localized using the lower frequency magnetic fields.

Yet in another embodiment, the MR and coils sensors connected to the same input channels on the control unit are not orientationally aligned and form a 6DOF sensor. In this case, the 6DOF sensor tracking capability is achieved with just a single input channel.

The control unit 315 is configured to collate MR sensor 210 and coil sensor 514 to provide a single set of position and orientation values.

In another embodiment, the MR sensor 210 and the coil sensor 514 are simultaneously connected to different control unit 315 input channels, as shown in FIG. 5. In this case, each sensor's inputs are processed through different channels both containing analog filters 332, amplifiers 335, ADC 340, and demodulation components 342. MR sensors respond directly to the magnetic field strength, while coil sensors respond to its time derivative.

Further, the antenna elements are energized by time-varying current creating sinusoidal magnetic fields. Each antenna element is energized by a current having a unique frequency $f_i$, i-1,...N. The responses generated by the MR sensors to such magnetic fields are directly proportional to the magnetic field strength, while responses generated by coil sensors are proportional to the time derivative of the magnetic field.

In another embodiment, two different sets of magnetic fields are created by each antenna element: one set with low frequencies and one set with high frequencies. The low and high frequency signals are processed in control unit 315 by applying the appropriate demodulation filters 342: low frequency for MR sensors and high frequency for coil sensors. Eddy currents are created by time varying magnetic fields in the surrounding conductors, which in turn generate magnetic fields causing distortion on the sensor inputs. The strength of the eddy currents is proportional to the time derivative of the magnetic field. The lower the frequency of the magnetic fields generated by antenna elements, the weaker the field generated by the eddy currents. For example, magnetic fields varying with 300 Hz will generate 30 times weaker eddy currents than magnetic fields varying with 9,000 Hz, leading to correspondingly smaller low frequency magnetic fields distortion.

In one embodiment, MR sensors inputs are demodulated on low frequencies, allowing low sensitivity to the eddy current distortion, while the coil sensors inputs are demodulated on the high frequencies. As an example, the low frequencies could be chosen in the range of 1Hz -1KHz, while high frequencies could be in the range of 9KHz-12KHz.

Yet in another embodiment, an MR sensor is placed in close proximity with a coil sensor within a tool, so that coil sensor positions and orientations having high frequency sampling rate could be corrected using the MR sensor positions and orientations having a low frequency sampling rate. For example, position and orientation sampling rates for the coil sensor can be as high as 100Hz, and as low as 10Hz for the MR sensor. In this embodiment, antenna elements are concurrently energized with two sets of frequencies: one set utilizing high frequency and another set utilizing low frequency. While coil sensor positions and orientations may be affected by distortion caused by eddy currents, the effect of eddy currents distortions on MR sensor positions and orientations will be much smaller. In this case, due to the low frequency of the magnetic fields used for tracking the MR sensor, the resulting location sampling rate for the MR sensor is also expected to be respectively low, while the location sampling rate for the co-located coil sensor is expected to be respectively high. However, being unaffected by the eddy currents, the MR sensor positions and orientations will be much more accurate, allowing correcting of the positions and orientations of the coil sensor. Using the slower sampling rate MR sensor to dynamically correct the higher sampling rate coil-based sensor will lead to better overall accuracy without sacrificing sensor responsiveness.

In one embodiment, the update of the sensor positions and orientations can be implemented as follows. Let $\boldsymbol{X}_i$ be a sensor position (for the collated MR and coil sensors) reported by the system at the time sample $i$, $\Delta\boldsymbol{X}_j$ - the distortion correction value, and $\boldsymbol{X}MR_j$, $\boldsymbol{X}C_j$ - slow and fast sample rate positions from the MR and coil sensors correspondingly, Let $\alpha$ be the exponential moving average filter characteristic constant, which determines the distortion correction value update rate. The value of $\alpha$ between 0 and 1 can be chosen according to specific tracking conditions and distortion patterns. For smooth tracking, $\alpha$ in the range of 0.1-0.5 can be used. To correct fast sampled coil sensor-derived positions and orientations using the slow-sampled MR sensor positions and orientations, the following adaptive filter on distortion parts of the coil-derived positions and orientations is used. For positions:

| initialize: | $\boldsymbol{X}_0 = \boldsymbol{X}C_0, \Delta X_0 = 0,$ | (2) |
|---|---|---|
| distortion update: | $\Delta\boldsymbol{X} = (1 - \alpha) \Delta\boldsymbol{X}_{j-1} + \alpha (\boldsymbol{X}MR_j - \boldsymbol{X}C_j),$ | (3) |
| location update: | $\boldsymbol{X}_i = \boldsymbol{X}C_i + \Delta\boldsymbol{X}_j,$ | (4) |

The $\boldsymbol{X}_i$ update rate in Equation 4 is the same as the fast update rate for the coil - derived positions.

The orientation correction of the collated sensor is performed as follows. In one embodiment, the directions of 5DOF sensors and three dimensional orientations of 6DOF sensors are described by quaternions, which are conventionally used to describe 3-dimensional rotations. All 5DOF sensor direction vectors, as well as 3D orientations describing 6DOF sensors, can be converted into the corresponding quaternions and visa versa using standard mathematical techniques. Let the coil sensor orientation time samples be described by the quaternions $\boldsymbol{q}C_i$, and the orientations of the MR sensor - by the quaternions $\boldsymbol{q}MR_j$. To calculate distortion corrections $\Delta\boldsymbol{q}_j$ to the orientation quaternions $\boldsymbol{q}_i$ of the collated sensor, the following algorithm is used:

| initialize: | $\boldsymbol{q}_0 = \boldsymbol{q}C_0, \Delta q_0 = (1,0,0,0)$ | (5) |
|---|---|---|
| distortion update: | $\Delta\boldsymbol{q}_j = (\boldsymbol{q}C_{j-1}^{-1*}\boldsymbol{q}MR_j)^\alpha$ | (6) |
| orientation update: | $\boldsymbol{q}_i = \boldsymbol{q}C_i * \Delta\boldsymbol{q}_j,$ | (7) |

The meaning of Equations (4) and (7) is that the distortion corrections $\Delta\boldsymbol{X}_j$ and $\Delta\boldsymbol{q}_j$ to the collated sensor positions and orientations $\boldsymbol{X}_i$ and $\boldsymbol{q}_i$ derived using the MR sensor positions and orientations stay the same between the slow sampled MR sensor positions $\boldsymbol{X}MR_j$ and orientations $\boldsymbol{q}MR_j$ but are used to continuously update $\boldsymbol{X}_i$ and $\boldsymbol{q}_i$ based on the fast sampled positions $\boldsymbol{X}C_i$ and orientations $\boldsymbol{q}C_i$ of the coil sensor. According to the Equations (3) and (6), these corrections are effectively filtered using the exponential moving average filter with the filter constant $\alpha$. The value $\alpha = 0$ corresponding to no distortion correction of the coil sensor using MR sensor position and orientation information, and $\alpha = 1$ corresponding to applying the full correction when new MR sensor position and orientation become available.

In another embodiment, shown in FIG 6, both the MR sensor 210 and coil sensor 514 are connected to the same input channel of the control unit 315 through conductors 330.

In another embodiment, the magnetic field is created in each antenna element by a current having sinusoidal waveform function such as $Sin(2\pi f_\alpha t)$, where $f_\alpha$ is the frequency of the current applied to $\alpha$-th antenna element. Then the MR sensor 210 response would also be proportional to $Sin(2\pi f_\alpha t)$, while the coil sensor 514 response would be proportional to its time derivative: $2\pi f_\alpha Cos(2\pi f_\alpha t)$, which can be mathematically equivalently represented as proportional to the phase shifted sine function: $2\pi f_\alpha Sin(2\pi f_\alpha t + \pi/2)$. The relative phase shift between the MR sensor 210 and coil sensor 514 signal outputs is used to separate the responses when both sensors are connected to the same input channel on the control unit 315.

FIG. 7 is a block diagram of a signal processing method 700 used to process the input waveform 710 from the sensors of tracking system 600. Two different digital filter banks 715 and 720 are used to localize the different sensors. In one embodiment, each filter bank contains N finite impulse response (FIR) filters with tap coefficients equal to the time samples of the appropriate cosine and sine functions respectively, where N is the number of antenna elements used to produce the electromagnetic field. Filter bank 715 uses a digital cosine FIR filter bank to generate coil sensor signals in-phase pickup components 725. Filter bank 720 uses a digital sine FIR filter bank to generate MR sensor quadrature pickup components 730. In another embodiment, infinite impulse response (IIR) filters could be used in a similar manner instead of FIR filters to produce the sensor pickup components 725 and 730. Each bank produces N pickups which are used to compute positions and orientations of coil sensor and MR sensor respectively. Localization of the respective sensors occurs at blocks 735 and 740 using Equations (1) and (1') or with commercially available localization software, including without limitations Radwave Technologies Inc. localization software.

Demodulation of the input waveform 710 occurs by applying the corresponding FIR filter to the digitized input signals. The FIR filter with coefficients sampled from the cosine function produces what is known as in-phase pickup components 725, and the FIR filter with coefficients sampled from the sine function produces what is known as quadrature pickup components 730. This approach is widely used with the conventional Orthogonal Frequency Division Multiplexing (OFDM) demodulation.

Let $y_i$ be signal samples on the output of the ADC 340, $i$ - the time sample number at which the sample is collected. Let the demodulation frame window contain M time samples. Let a filter bank corresponding to the antenna frequency $2\pi f_\alpha$, $\alpha$ =1,...,N, have the following tap coefficients: $a_{\alpha i} = Cos(2\pi f_\alpha t_i)$ for the filter producing in-phase components and $b_{\alpha i} = Sin(2\pi f_\alpha t_i)$ for the filter producing quadrature components, Here i=1,...,M, is the size of the demodulation frame such that all filter taps are mutually orthogonal to each other, $t_i$=i T/M, where T is the time length of the demodulation window. In the OFDM approach, T is chosen to contain a whole number of periods of each of the M waveforms to ensure the mutual orthogonality of the waveforms within the window. In this embodiment, only a relatively small number of possible orthogonal waveforms are used, which allows us to effectively use the FIR and IIR filters instead of the full inverse Fourier transforms used in the conventional OFDM applications.

The pickups $V_\alpha$ corresponding to the in-phase components are computed as

$$V_\alpha = \sum_{i=1}^{M} y_i \; a_{\alpha i},$$

and pickups $W_\alpha$ corresponding to the quadrature components are computed as

$$W_\alpha = \sum_{i=1}^{M} y_i \; b_{\alpha i}.$$

Let antenna elements be energized with the sine function waveform. Then the MR sensor response will have a sine function waveform, and the coil sensor response will have a cosine function waveform (due to the time derivative response of the coil sensor). If both sensors output voltages are summed and provided to the same channel of the control unit, their respective input waveforms corresponding to the same frequencies will be shifted in phase by 90 degrees (or equivalently, by $\pi/2$) to each other. Thus, the input waveforms will be separated by the two filter banks into two different sets of pickups: $V_\alpha$ for the coil sensor and $W_\alpha$ for the MR sensor, allowing simultaneous and independent localization of both sensors.

FIG. 8 is a flowchart illustrating method 800 for performing dynamic range adjustment on using control unit pickups output 810. One potential problem when tracking the MR sensors is their limited dynamic range, limiting the tracking volume within which the sensor can be tracked by an electromagnetic tracking system. In one embodiment, to extend the MR sensor dynamic range, an automatic MR sensor dynamic range adjustment is applied. To automatically adjust the dynamic range of a sensor, low and high thresholds are established. The RMS (root-mean-square) of the sensor pickups is computed as RMS(sensor pickups) = $\sqrt{((V_1^2 + V_2^2 + ... + V_N^2)/N)}$, where $V_\alpha$ is the input pickup voltage amplitude from the $\alpha$-th antenna element, N is the number of antenna elements. The thresholds are set up so that the sensor's output signal is sufficiently strong to enable reliable tracking when the RMS(sensor pickups) is between the thresholds. Alternatively, the absolute value of the sensor pickup can be used instead of the RMS value.

The sensitivity of an MR sensor is typically measured in Volts/Volts/Tesla because its response is dependent on the power input voltage provided to the sensor. To extend the sensor's dynamic range, the power input voltage can be controlled to cause the MR sensor effective sensitivity, measured in Volts/Tesla (output voltage produced for a given magnetic field strength measured), to be adjusted up or down by the corresponding adjustment of the power input voltage 313 or 324 in FIG. 3, 5 and 6.

If the MR sensor is too high in the tracking volume so that the sensor output signal becomes too weak, as determined at decision block 815 where the RMS is less than V_low threshold, the power input voltage to the sensor is increased at operation 840, and the sensor gain value used within the localization Equations (1) and (1') is correspondingly increased at operation 845. These operations increase the effective sensitivity of the sensor.

If, on the other hand, the MR sensor is too low in the tracking volume, meaning it is too close to the antenna, the strong magnetic fields generated by antenna elements may saturate it, thus distorting the sensor output signal. To make the sensor less sensitive to the magnetic field, a V_high threshold is used within the decision block 820. If V_high threshold is less than the RMS value at decision block 820, the sensor power input voltage is decreased at operation 825 and the sensor gain value in the localization Equations (1) and (1') is correspondingly decreased at operation 830, which reduces the effective sensitivity of the sensor.

If V_high threshold is not less than the RMS value at decision block 820, no change is made to the sensor power input voltage at operation 835.

This process is performed on each set of pickups produced by the control unit output 810.

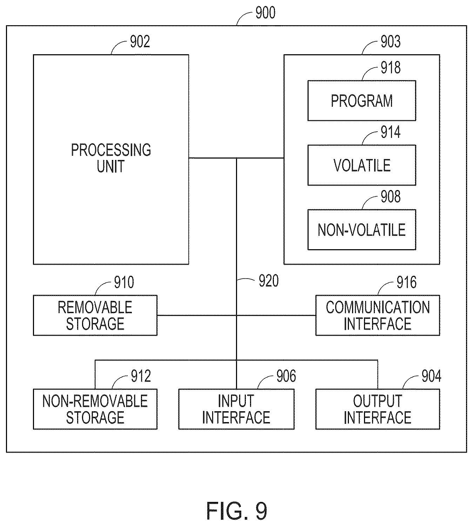

FIG. 9 is a block schematic diagram of a computer system 900 to implement a controller for MR sensor-based position sensing and for performing methods and algorithms according to example embodiments. All components need not be used in various embodiments.

One example computing device in the form of a computer 900 may include a processing unit 902, memory 903, removable storage 910, and non-removable storage 912. Although the example computing device is illustrated and described as computer 900, the computing device may be in different forms in different embodiments. For example, the computing device may instead be a smartphone, a tablet, smartwatch, smart storage device (SSD), or other computing device including the same or similar elements as illustrated and described with regard to FIG. 9. Devices, such as smartphones, tablets, and smartwatches, are generally collectively referred to as mobile devices or user equipment.

Although the various data storage elements are illustrated as part of the computer 900, the storage may also or alternatively include cloud-based storage accessible via a network, such as the Internet or server-based storage. Note also that an SSD may include a processor on which the parser may be run, allowing transfer of parsed, filtered data through I/O channels between the SSD and main memory.

Memory 903 may include volatile memory 914 and non-volatile memory 908. Computer 900 may include - or have access to a computing environment that includes - a variety of computer-readable media, such as volatile memory 914 and non-volatile memory 908, removable storage 910 and non-removable storage 912. Computer storage includes random access memory (RAM), read only memory (ROM), erasable programmable read-only memory (EPROM) or electrically erasable programmable read-only memory (EEPROM), flash memory or other memory technologies, compact disc read-only memory (CD ROM), Digital Versatile Disks (DVD) or other optical disk storage, magnetic cassettes, magnetic tape, magnetic disk storage or other magnetic storage devices, or any other medium capable of storing computer-readable instructions.

Computer 900 may include or have access to a computing environment that includes input interface 906, output interface 904, and a communication interface 916. Output interface 904 may include a display device, such as a touchscreen, that also may serve as an input device. The input interface 906 may include one or more of a touchscreen, touchpad, mouse, keyboard, camera, one or more device-specific buttons, one or more sensors integrated within or coupled via wired or wireless data connections to the computer 900, and other input devices. The computer may operate in a networked environment using a communication connection to connect to one or more remote computers, such as database servers. The remote computer may include a personal computer (PC), server, router, network PC, a peer device or other common data flow network switch, or the like. The communication connection may include a Local Area Network (LAN), a Wide Area Network (WAN), cellular, Wi-Fi, Bluetooth, or other networks. According to one embodiment, the various components of computer 900 are connected with a system bus 920.

Computer-readable instructions stored on a computer-readable medium are executable by the processing unit 902 of the computer 900, such as a program 918. The program 918 in some embodiments comprises software to implement one or more methods described herein. A hard drive, CD-ROM, and RAM are some examples of articles including a non-transitory computer-readable medium such as a storage device. The terms computer-readable medium, machine readable medium, and storage device do not include carrier waves or signals to the extent carrier waves and signals are deemed too transitory. Storage can also include networked storage, such as a storage area network (SAN). Computer program 918 may be used to cause processing unit 802 to perform one or more methods or algorithms described herein.

Examples:

1. An electromagnetic tracking system includes an adjustable source of electricity, a magneto-resistive (MR) sensor coupled to the adjustable source of electricity and configured to couple to a device for tracking a position of the device, and a controller coupled to the adjustable source of electricity and the MR sensor to adjust an amount of electricity provided to the MR sensor by the adjustable source of electricity.

2. The system of example 1 where the adjustable source of electricity provides electricity to the MR sensor in the form of a voltage that is scalable by the controller to adjust a sensitivity of the MR sensor.

3. The system of example 2 wherein the controller controls the voltage to increase an effective dynamic range of the sensor in areas of strong magnetic fields by reducing the voltage and increases the voltage in areas of weak magnetic fields.

4. The system of any of examples 1-3 and further including a coil-based sensor coupled to the controller and wherein the controller is configured to localize the coil-based sensor and MR sensor at the same time.

5. The system of example 4 wherein the controller adjusts a position and orientation sampling rate of the MR sensor to be lower than a coil sensor position and orientation sampling rate.

6. The system of any of examples 4-5 wherein the controller is configured to collate the MR and coil sensors to provide a common application programming interface that abstracts a sensor type from a client tracking application.

7. The system of any of examples 4-6 and further including a magnetic field generator configured to generate magnetic fields at typical frequencies used for a coil-based sensor such that both coil-based and MR sensors are trackable using the same magnetic fields at the same time.

8. The system of example 7 wherein the controller is configured to receive signals from both coil-based sensor types and MR sensor types by localizing one sensor type using in-phase demodulation channel information and localizing the other sensor type using quadrature demodulation channel information.

9. The system of any of examples 4-8 wherein the controller is configured to recognize coil-based sensors and MR sensors and to set up the sensors for tracking based on information stored in a pre-configured sensor memory.

10. The system of any of examples 4-9 whereon the controller is configured to localize the MR sensor using low or zero frequency magnetic fields to minimize or remove magnetic field distortions caused by eddy currents produced in nearby conductive materials.

11. The system of example 10 wherein the MR sensor and coil sensor are coupled to the adjustable source of electricity by a same physical input channel.

12. The system of example 11 wherein the controller is configured to localize the sensors based on different demodulated components of signals received from the sensors on the same channels.

13. The system of any of examples 4-12 and further comprising a hybrid sensor assembly that includes the coil-based sensor comprising a five degree of freedom (5DOF) sensor and the MR sensor comprising a 5DOF MR sensor having its sensitivity axis mis-aligned from the coil-based sensor sensitivity axis to produce a single six degree of freedom (6DOF) sensor.

14. The sensor of example 13 wherein the 6DOF sensor utilizes a single input channel to produce a 6DOF sensor position and orientation signal.

15. The system of any of examples 10-14 and further comprising a hybrid sensor assembly that includes the coil-based sensor comprising a 5DOF sensor and the MR sensor comprising a co-located 5DOF MR sensor that are aligned to produce a sensor 5DOF sensor position and orientation signal, wherein the coil-based sensor is localized using higher frequency magnetic fields and the MR sensor is localized using lower or zero frequency magnetic fields, and wherein the controller is configured to use the position and orientation signal of the MR sensor to correct the position and orientation of the coil sensor.

16. The system of any of examples 1-15 and further comprising a feedback loop using a temperature sensor coupled to MR sensor and to the controller to improve temperature stability of the MR sensor, wherein the controller is configured to adjust an amount of electricity provided by regulating an input power voltage supplied to the MR sensor as a function of sensed temperature.

17. The system of any of examples 1-16 wherein the controller is configured to perform a localization algorithm based on relative magnetic field signal levels instead of absolute magnetic field signal levels to compute the MR sensor effective sensitivity at the same time as position and orientation to provide temperature independence of the sensor.

18. A method includes providing electricity to a magneto-resistive (MR) sensor configured to couple to a device for tracking a position of the device, measuring a magnitude of a magnetic field at the MR sensor, and controlling an input voltage provided to the MR sensor based on the measured magnitude to adjust the dynamic range of the MR sensor.

19. The method of example 18, wherein the electricity provided to the MR sensor is in the form of an adjustable input voltage.

20. The method of any of examples 18-19, further comprising measuring an output signal of the MR sensor to determine the magnitude of the magnetic field.

21. The method of any of examples 18-22, further comprising computing a root-mean-square (RMS) value of an output signal of the MR sensor to determine strength of the MR sensor output signal.

22. The method of any of examples 18-21, wherein controlling the input voltage comprises increasing the input voltage in response to the strength of the output signal of the MR sensor falling below a predetermined threshold.

23. The method of any of examples 18-22, wherein controlling the amount of electricity comprises decreasing the input voltage in response to the strength of an output signal of the MR sensor exceeding a predetermined threshold.

24. The method of any of examples 18-23, wherein the adjustment of the electricity provided to the MR sensor is performed automatically by a controller.

25. The method of any of examples 18-24, further comprising regulating a temperature of the MR sensor to improve stability of an output of the sensor.

26. The method of any of examples 18-25, wherein the MR sensor is co-located with a coil sensor, and the method further comprises tracking the position of the device using both the MR sensor and the coil sensor.

27. The method of any of examples 18-26, wherein the adjustment of the electricity provided to the MR sensor is performed in real time.

28. The method of any of examples 18-27, wherein the MR sensor is configured to operate within a time-division-multiplexing scheme for energizing antenna elements of an electromagnetic field generator.

The functions or algorithms described herein may be implemented in software in one embodiment. The software may consist of computer executable instructions stored on computer readable media or computer readable storage device such as one or more non-transitory memories or other type of hardware-based storage devices, either local or networked. Further, such functions correspond to modules, which may be software, hardware, firmware or any combination thereof. Multiple functions may be performed in one or more modules as desired, and the embodiments described are merely examples. The software may be executed on a digital signal processor, ASIC, microprocessor, or other type of processor operating on a computer system, such as a personal computer, server or other computer system, turning such computer system into a specifically programmed machine.

The functionality can be configured to perform an operation using, for instance, software, hardware, firmware, or the like. For example, the phrase "configured to" can refer to a logic circuit structure of a hardware element that is to implement the associated functionality. The phrase "configured to" can also refer to a logic circuit structure of a hardware element that is to implement the coding design of associated functionality of firmware or software. The term "module" refers to a structural element that can be implemented using any suitable hardware (e.g., a processor, among others), software (e.g., an application, among others), firmware, or any combination of hardware, software, and firmware. The term, "logic" encompasses any functionality for performing a task. For instance, each operation illustrated in the flowcharts corresponds to logic for performing that operation. An operation can be performed using, software, hardware, firmware, or the like. The terms, "component," "system," and the like may refer to computer-related entities, hardware, and software in execution, firmware, or combination thereof. A component may be a process running on a processor, an object, an executable, a program, a function, a subroutine, a computer, or a combination of software and hardware. The term, "processor," may refer to a hardware component, such as a processing unit of a computer system.

Furthermore, the claimed subject matter may be implemented as a method, apparatus, or article of manufacture using standard programming and engineering techniques to produce software, firmware, hardware, or any combination thereof to control a computing device to implement the disclosed subject matter. The term, "article of manufacture," as used herein is intended to encompass a computer program accessible from any computer-readable storage device or media. Computer-readable storage media can include, but are not limited to, magnetic storage devices, e.g., hard disk, floppy disk, magnetic strips, optical disk, compact disk (CD), digital versatile disk (DVD), smart cards, flash memory devices, among others. In contrast, computer-readable media, i.e., not storage media, may additionally include communication media such as transmission media for wireless signals and the like.

Although a few embodiments have been described in detail above, other modifications are possible. For example, the logic flows depicted in the figures do not require the particular order shown, or sequential order, to achieve desirable results. Other steps may be provided, or steps may be eliminated, from the described flows, and other components may be added to, or removed from, the described systems. Other embodiments may be within the scope of the following claims.

**Claims**

1.  An electromagnetic tracking system (300, 500, 600) comprising:

    an adjustable source of electricity (313);
    a magneto-resistive (MR) sensor (210) coupled to the adjustable source of electricity and configured to couple to a device (144) for tracking a position of the device; and
    a controller (315) coupled to the adjustable source of electricity and the MR sensor to adjust an amount of electricity provided to the MR sensor by the adjustable source of electricity.

2.  The system of claim 1 where the adjustable source of electricity provides electricity to the MR sensor in the form of a voltage that is scalable by the controller to adjust a sensitivity of the MR sensor.

3.  The system of claim 2 wherein the controller controls the voltage to increase an effective dynamic range of the sensor in areas of strong magnetic fields by reducing the voltage and increasing the voltage in areas of weak magnetic fields.

4.  The system of claim 1 and further comprising a feedback loop (355) using a temperature sensor (350) coupled to MR sensor and to the controller to improve temperature stability of the MR sensor, wherein the controller is configured to adjust an amount of electricity provided by regulating an input power voltage supplied to the MR sensor as a function of sensed temperature.

5.  The system of any one of claims 1-4 and further comprising:
    a coil-based sensor (514) coupled to the controller and wherein the controller is configured to localize the coil-based sensor and MR sensor.

6.  The system of claim 5 wherein the controller adjusts a position and orientation sampling rate of the MR sensor to be lower than a coil-based sensor position and orientation sampling rate.

7.  The system of claim 4 wherein the controller is configured to collate the MR sensor and coil-based sensor to provide a common application programming interface that abstracts a sensor type from a client tracking application.

8.  The system of claim 4 and further comprising a magnetic field generator (142) configured to generate magnetic fields at frequencies used for a coil-based sensor such that both coil-based and MR sensors are trackable using the same magnetic fields at the same time.

9.  The system of claim 8 wherein the controller is configured to receive signals from both coil-based sensor types and MR sensor types by localizing one sensor type using in-phase demodulation channel information and localizing the other sensor type using quadrature demodulation channel information.

10. The system of any one of claims 1-4 and further comprising a hybrid sensor assembly (608) that includes the coil-based sensor comprising a five degree of freedom (5DOF) sensor and the MR sensor comprising a 5DOF MR sensor having its sensitivity axis mis-aligned from the coil-based sensor sensitivity axis to produce a single six degree of freedom (6DOF) sensor.

11. The sensor of claim 10 wherein the 6DOF sensor utilizes a single input channel to produce a 6DOF sensor position and orientation signal.

12. The system of any one of claims 1-4 and further comprising a hybrid sensor assembly that includes the coil-based sensor comprising a 5DOF sensor and the MR sensor comprising a co-located 5DOF MR sensor that are aligned to produce a sensor 5DOF sensor position and orientation signal, wherein the coil-based sensor is localized using higher frequency magnetic fields and the MR sensor is localized using lower or zero frequency magnetic fields, and wherein the controller is configured to use the position and orientation signal of the MR sensor to correct the position and orientation of the coil-based sensor.

13. A method (800) comprising:

    providing (810) electricity to a magneto-resistive (MR) sensor configured to couple to a device for tracking a position of the device;

measuring (815) a magnitude of a magnetic field at the MR sensor; and
controlling (840, 825, 835) an input voltage provided to the MR sensor based on the measured magnitude to adjust a dynamic range of the MR sensor.

14. The method of claim 13, controlling the input voltage comprises decreasing (825) the input voltage in response to an output signal of the MR sensor exceeding a predetermined threshold, and further comprising computing a root-mean-square (RMS) value of an output signal of the MR sensor to determine strength of the MR sensor output signal.

15. The method of any one of claims 12-13, wherein the MR sensor is co-located (608) with a coil sensor, and the method further comprises tracking the position of the device using both the MR sensor and the coil sensor.

FIG. 1

200

CHANGE IN RESISTANCE ACROSS
MAGNETORESISTANCE MATERIAL

207

MAGNETORESISTANCE
MATERIAL

205

213

211

V 209

FIG. 2A

210

MAGNETO RESISTIVE SENSOR INTEGRATED CIRCUIT
(HALF BRIDGE CONFIGURATION)

211

205 MAGNETO RESISTIVE MATERIAL

207

FIXED RESISTANCE

CHANGE IN RESISTANCE ACROSS MAGNETO RESISTIVE MATERIAL

FIXED RESISTANCE

MAGNETO RESISTIVE MATERIAL 205

207

213

$V_{IN}^+$

$V_{OUT}^+$

$V_{IN}^-$

$V_{OUT}^-$

FIG. 2B

FIG. 2C

FIG. 2D

FIG. 3

FIG. 4

FIG. 5

FIG. 6

735 LOCALIZE COIL SENSOR

740 LOCALIZE MR SENSOR

725 COIL SENSOR PICKUPS

730 MR SENSOR PICKUPS

715 DIGITAL COS FIR FILTER BANK CONTAINING N FILTERS (IN-PHASE)

720 DIGITAL SINE FIR FILTER BANK CONTAINING N FILTERS (QUADRATURE)

710 INPUT WAVEFORM

700

FIG. 7

FIG. 8

FIG. 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63711833